# EUROPÄISCHE PATENTSCHRIFT

(11) **EP 1 366 362 B1**
(45) Veröffentlichungstag und Bekanntmachung des Hinweises auf die Patenterteilung: **16.11.2005**
(21) Anmeldenummer: 01988862.7
(22) Anmeldetag: 26.10.2001
(51) Int. Cl.: G01N 33/50, G01N 33/569

(54) **VERFAHREN ZUM NACHWEIS APOPTOTISCHER UND NEKROTISCHER ZELLEN**
METHOD FOR THE IDENTIFICATION OF APOPTOTIC AND NECROTIC CELLS
PROCEDE D'IDENTIFICATION DE CELLULES APOPTOTIQUES ET NECROTIQUES

(30) Priorität: 27.10.2000 DE 10053521
(43) Veröffentlichungstag der Anmeldung: 03.12.2003
(73) Patentinhaber: responsif GmbH, 91056 Erlangen (DE)
(72) Erfinder: HERRMANN, Martin, 91077 Neunkirchen (DE); KALDEN, Joachim, Robert, 91054 Erlangen (DE); BERTLING, Wolf, 91056 Erlangen (DE); REISER, Christian, 96047 Bamberg (DE); GAIPL, Udo, 91058 Erlangen (DE); HEYDER, Petra, 90592 Schwarzenbruck (DE); WOITH, Walter, 91056 Erlangen (DE)
(74) Vertreter: Gassner, Wolfgang
(86) Internationale Anmeldenummer: PCT/DE2001/004028
(87) Internationale Veröffentlichungsnummer: WO 2002/035229

(56) Entgegenhaltungen:
- WO-A-98/11439
- KULKARNI G V ET AL: "Concanavalin A induced apoptosis in fibroblasts: The role of cell surface carbohydrates in lectin mediated cytotoxicity." JOURNAL OF CELLULAR PHYSIOLOGY, Bd. 165, Nr. 1, 1995, Seiten 119-133, XP008003171 ISSN: 0021-9541 in der Anmeldung erwähnt

## Beschreibung

Die Erfindung betrifft ein Verfahren zum Nachweis apoptotischer und nekrotischer Zellen in einer Zellsuspension.

Aus der WO 98/11439 A1 ist es zum Nachweis apoptotischer Zellen bekannt, die Zellen mit einer Substanz zu behandeln, welche die Zytoplasmamembran der Zellen permeabilisiert. Diese Substanz kann beispielsweise Ethanol sein. Aus den so permeabilisierten Zellen werden die durch Apoptose erzeugten DNA-Fragmente extrahiert und die noch vorhandene zelluläre DNA durch einen geeigneten Marker, wie z.B. Propidiumiodid, markiert. Anschließend erfolgt ein Nachweis der Ploidie der Zellen mittels Durchflußzytometrie.

Aus Reutelingsperger, C.P. et al., Cell. Mol. Life Sci. 53 (1997) Seiten 527 - 532 ist es bekannt, daß apoptotische und nekrotische Zellen Phosphatidylserin auf der äußeren Zellmembran exponieren. Apoptotische und nekrotische Zellen können durch die Bindung des Phosphatidylserin-bindenden Proteins Annexin V auf der Zelloberfläche nachgewiesen werden. Dazu kann Annexin V mit einem Fluoreszenzfarbstoff markiert sein. Eine dadurch hervorgerufene Färbung apoptotischer und nekrotischer Zellen ist nur für 4 bis 5 Stunden stabil. Der eindeutige Nachweis apoptotischer Zellen gelingt bei diesem Verfahren,in Abhängigkeit vom Induktor erst einige Stunden nach einem Apoptose-auslösenden Ereignis. Das Apoptose-auslösende Ereignis kann eine UV- oder γ-Bestrahlung oder eine Behandlung mit Staurosporin oder mit gleichwirkenden Agentien sein.

Aus Gorczyca, W., Endocrine-Related Cancer (1999) 6, 19 - 17 ist es bekannt, apoptotische Zellen unter Verwendung von Annexin V-FITC nachzuweisen.

Die Zellmembran apoptotischer Zellen ist im Gegensatz zu derjenigen nekrotischer Zellen intakt. Es ist allgemein bekannt, dieses Phänomen zur Unterscheidung zwischen apoptotischen und nekrotischen Zellen zu nutzen. Apoptotische Zellen werden durch einen das Zellinnere, insbesondere den Zellkern, anfärbenden Farbstoff, wie Propidiumiodid, wesentlich langsamer gefärbt als nekrotische Zellen.

Aus Falasca, L. et al., Exp. Cell Res. (1996), Seiten 152 - 162 ist es bekannt, daß die äußeren Zellmembranen apoptotischer peripherer Blutlymphozyten erhöhte Mengen von N-Acetylgalactosamin, D-Galactose und Mannose-Reste im Vergleich zu äußeren Zellmembranen normaler peripherer Blutlymphozyten exponieren. Die erhöhte Exposition dieser Kohlenhydrate auf der Oberfläche apoptotischer Zellen kann durch Bindung der Lektine Concanavalin A, Ulex europaeus I und Phaseolus limensis nachgewiesen werden.

Kulkarni, G.W. & McCulloch, C.A., Journal of Cellular Physiology, (Oktober 1995) 165 (1) 119 - 133 beschreibt Untersuchungen zu Funktion von Kohlehydraten auf der Zelloberfläche bei der Apoptose. Daraus ist es auch bekannt, daß Concanavalin A in der Lage ist, an die Oberfläche von nichtapoptotischen Fibroblasten zu binden und nach einigen Stunden die Apoptose zu induzieren.

Boldt, D.H., Journal of Cellular Physiology, (Juli 1984) 120 (1) 61-1 und Blackledge, G. u.a., Biochemical Journal, (15. Oktober 1982) 208 (1) 69 - 75 beschreiben die Verwendung von Lektinen zur Unterscheidung verschiedener Subpopulationen von Lymphozyten. Bei diesen Lymphozyten handelt es sich jedoch nicht um apoptotische Zellen.

Zur durchflußzytometrischen Analyse von in einer Blutprobe enthaltenen Leukozyten ist es allgemein bekannt, der Blutprobe Antikörper zuzusetzen, die an bestimmte Oberflächenantigene der Leukozyten binden. Anschließend wird die Blutprobe mit Säure behandelt..Dabei lysieren die Erythrozyten, während die Leukozyten intakt bleiben. Nach der Lyse der Erythrozyten wird der pH-Wert der Blutprobe wieder neutralisiert. Danach können die Leukozyten mittels einer Paraformaldehydlösung fixiert werden. Die an der Oberfläche der Leukozyten gebundenen Antikörper können durchflußzytometrisch nachgewiesen werden. Das Verfahren kann automatisiert mittels des Coulter® Q-Prep®-Sytems durchgeführt werden.

Aufgabe der Erfindung ist es, ein alternatives Verfahren bereitzustellen, mit dem kurze Zeit nach einem Apoptose-auslösenden Ereignis apoptotische und nekrotische Zellen nachgewiesen werden können. Ferner soll ein Kit bereitgestellt werden, mit dem das Verfahren durchgeführt werden kann.

Diese Aufgabe wird durch die Merkmale der Ansprüche 1 und 15 gelöst. Zweckmäßige Ausgestaltungen der Erfindung ergeben sich aus den Merkmalen der Ansprüche 2 bis 14 sowie 16 bis 20.

Zur Lösung der Aufgabe ist ein Verfahren zum Nachweis apoptotischer und nekrotischer Zellen in einer Zellsuspension mit den folgenden Schritten vorgesehen:
a) Inkontaktbringen einer der Zellsuspension entnommenen Probe mit einem Lektin, welches dazu geeignet ist, an in der Membran apoptotischer und nekrotischer Zellen enthaltene Zuckerstrukturen zu binden,
b) Ansäuern der Probe, wobei eine solche Menge an Säure zugegeben wird, daß dadurch die äußere Zellmembran apoptotischer Zellen permeabilisiert wird, während die Zellmembran vitaler Zellen nicht permeabilisiert wird und
c) Detektieren der Menge des an apoptotische und nekrotische Zellen gebundenen Lektins.

Die Zellsuspension kann eine natürlich vorkommende Zellsuspension, wie Blut, sein. Es kann sich dabei auch um eine künstlich hergestellte, z.B. kultivierte oder aus Organen isolierte Zellen enthaltende, Zellsuspension handeln. Unter einem Lektin im Sinne der Erfindung wird jeder Stoff verstanden, der spezifisch an bestimmte Zuckerstrukturen binden kann. Das Ansäuern kann durch Zusetzen einer Säure oder einer im Saueren gepufferten Lösung erfolgen. Die Schritte lit. a) und lit. b) können in beliebiger Reihenfolge oder gleichzeitig durchgeführt werden. Zur gleichzeitigen Durchführung kann das Lektin in der Säure oder der im Saueren gepufferten Lösung enthalten sein. Das Detektieren der Menge des an apoptotische und nekrotische Zellen gebundenen bzw. zellgebundenen Lektins kann z.B. durch direkte Markierung oder mittels gegen das Lektin gerichteten markierten Antikörpern erfolgen. Es ist möglich, die Menge des gebundenen Lektins für jede Zelle einzeln zu bestimmen. Das kann z.B. mittels der Durchflußzytometrie erfolgen. Mittels Referenz-Suspensionen vitaler, apoptotischer und nekrotischer Zellen kann ein erster Schwellenwert für die Menge des von apoptotischen und nekrotischen Zellen gebundenen Lektins ermittelt werden. Zellen der Probe, bei denen die Menge des gebundenen Lektins den ersten Schwellenwert überschreitet, werden als apoptotische oder nekrotische Zellen erfaßt. Der Anteil apoptotischer und nekrotischer Zellen in der Probe kann näherungsweise mittels der Menge des insgesamt von allen Zellen in der Probe gebundenen Lektins ermittelt werden.

Das erfindungsgemäße Verfahren erlaubt bereits 5 - 10 Stunden nach einem Apoptose-auslösenden Ereignis den zuverlässigen Nachweis der apoptotischen und nekrotischen Zellen. Das Verfahren läßt sich relativ schnell durchführen.

Es hat sich überraschenderweise herausgestellt, daß die äußere Zellmembran apoptotischer Zellen durch Zugabe einer vorgegebene Menge an Säure angegriffen und dabei permeabilisiert wird. Zuckerstrukturen auf der Innenseite der Zellmembran apoptotischer Zellen werden durch das Ansäuern gemäß Schritt lit. b) für die Bindung des Lektins früher zugänglich. Für das Binden des Lektins an nekrotische Zellen ist die Säurebehandlung nicht erforderlich. Die vorgegebenen Menge an Säure ist stets so gewählt, daß die Zellmenbran vitaler Zellen nicht angegriffen und permeabilisiert wird. Das Ansäuern der Zellsuspension eröffnet eine Möglichkeit zur frühen Unterscheidung vitaler Zellen von apoptotischen. Zum Detektieren gemäß Schritt lit. c muß die Apoptose in den Zellen noch nicht so weit fortgeschritten sein, daß auf Grund der Destabilisierung der Zellmembran ursprünglich auf der Innenseite befindliche Zuckerstrukturen auf der Außenseite der Zelle präsentiert werden.

Das erfindungsgemäße Verfahren erlaubt einen Nachweis apoptotischer und nekrotischer Zellen in einem, insbesondere automatisiert durchführbaren, Routineverfahren, z.B. in der medizinischen Diagnostik.

Vorteilhafterweise wird der Probe beim Schritt lit. b) Ameisensäure oder Essigsäure zugesetzt. Ameisensäure und Essigsäure sind besonders gut geeignet, um die Bindung des Lektins an apoptotische Zellen früh nach einem Apoptose-auslösenden Ereignis zu ermöglichen ohne die Zellen zu fragmentieren.

Vorzugsweise wird der pH-Wert der angesäuerten Probe nach den Schritten lit. a) und lit. b) und vor dem Schritt lit. c) erhöht. Das kann erforderlich sein, wenn beim Schritt lit. b) in der Probe ein pH-Wert erreicht worden ist, der die Zellen bei längerer Einwirkung zerstören würde oder bei dem das spezifische Binden des Lektins nicht möglich ist. Ein Neutralisieren des pH-Werts ist nicht erforderlich. Es ist ausreichend wenn ein pH-Wert erreicht wird, bei dem die Zellen stabil sind und das Lektin spezifisch binden kann. Zum Erhöhen des pH-Werts kann der angesäuerten Probe ein Puffer, insbesondere ein Phosphatpuffer, oder eine alkalische Lösung, insbesondere eine Carbonatlösung, zugesetzt werden. Das Zusetzen eines Puffers hat gegenüber dem Zusetzen einer alkalischen Lösung den Vorteil, daß dadurch verhindert werden kann, daß die Probe zu stark alkalisch wird.

Bevorzugt enthalten die Zuckerstrukturen Mannosyl-, insbesondere α-D-Mannosyl-, Glucosyl-, insbesondere α-D-Glucosyl-, Glucosamin-, insbesondere N-Acetylglucosamin, Galactosyl-, Glactosamin-, insbesondere N-Acetylgalactosamin-, Fucosyl-, insbesondere L-Fucosyl-, oder N,N'-Diacetylchitobiose-Reste. Das Lektin kann Pisum sativum, Phaseolus limensis, Ulex europaeus I, Ulex europaeus II, Narcissus pseudonarcissus, Concanavalin A oder Ricinus communis sein. Ulex europaeus I bindet spezifisch an α-L-Fucosyl-Reste und Ulex europaeus II an N,N'-Diacetylchitobiose.

Das Lektin kann eine Markierungssubstanz aufweisen. Das ermöglicht ein Detektieren des zellgebundenen Lektins, ohne daß das Lektin mittels einer weiteren Substanz, wie einem Antikörper, nachgewiesen werden muß. Die Markierungssubstanz kann ein Fluorophor, insbesondere Fluoresceinisothiocyanat, sein.

Vorteilhafterweise erfolgt im unmittelbaren Anschluß an die Schritte lit. a) und lit. b) oder das Erhöhen des pH-Werts der angesäuerten Probe eine Fixierung der Zellen. Die Fixierung kann mittels Paraformaldehyd erfolgen. "Im unmittelbaren Anschluß" bedeutet, daß die Fixierung vor Schritt lit. c) zu einem Zeitpunkt erfolgt, zu dem sich die Menge des zellgebundenen Lektins gegenüber der Menge.des ursprünglich zellgebundenen Lektins noch nicht wesentlich verändert hat. Dadurch ist es möglich, den Schritt lit. c) bis zu 24 Stunden nach der Fixierung durchzuführen.

Zur Differenzierung zwischen apoptotischen und nekrotischen Zellen können die folgenden Schritte durchgeführt werden:
aa) Inkontaktbringen der Probe mit einem Stoff zur Anfärbung des Zellinneren, insbesondere des Zellkerns, welcher die äußere Zellmembran apoptotischer Zellen nicht oder langsam durchdringt und
bb) Detektieren der Menge des in das Innere der Zellen eingedrungenen Stoffs.

Der Nachweis apoptotischer und nekrotischer Zellen gemäß Schritt lit. a) und lit. b) kann dann im gleichen Behälter erfolgen wie die Differenzierung zwischen apoptotischen und nekrotischen Zellen gemäß Schritt lit. aa), wenn die Differenzierungsfärbung vor der Nachweisfärbung (lit. a bis lit. b) durchgeführt wird und dazwischen der überschüssige Stoff zur Anfärbung des Zellinneren durch Waschen entfernt wird.

Vorteilhafterweise werden die Schritte lit. c) und lit. bb) in einer gemeinsamen Messung durchgeführt. Das Verfahren macht sich die Tatsache zunutze, daß die äußere Zellmembran nekrotischer Zellen permeabel und die Zellmembran apoptotischer Zellen ohne Säurebehandlung für bestimmte Stoffe im wesentlichen nicht permeabel ist. Mittels einer Referenz-Suspension nekrotischer Zellen kann ein zweiter Schwellenwert für die Menge des in das Innere der Zellen eingedrungenen Stoffs ermittelt werden. Zellen der Probe, bei denen die Menge des in das Innere der Zelle eingedrungenen Stoffs den zweiten Schwellenwert übersteigt, werden als nekrotische Zellen erfaßt. Über den ersten Schwellenwert Lektin-bindende Zellen, bei denen die Menge des in das Innere der Zelle eingedrungenen Stoffs den zweiten Schwellenwert nicht übersteigt, sind apoptotisch. Der Anteil der Zellen, in die der Stoff eingedrungen ist, entspricht dem Anteil nekrotischer Zellen. Der Anteil nekrotischer Zellen in der Probe kann näherungsweise mittels der Menge des insgesamt von allen Zellen in der Probe aufgenommenen Stoffs ermittelt werden. Die Differenz zwischen dem mittels der Schritte lit. a) bis lit. c) ermittelten Anteil insgesamt vorhandener nekrotischer und apoptotischer Zellen und dem Anteil nekrotischer Zellen stellt den Anteil apoptotischer Zellen dar. Vorteilhafterweise ist der Stoff Propidiumiodid oder Trypanblau. Propidiumiodid färbt den Zellkern und ist durch seine intensive Fluoreszenz nachweisbar.

Vorzugsweise erfolgt bei einem Verfahren zur Identifikation nekrotischer Zellen zwischen den Schritten lit. aa) und lit. bb) ein Waschschritt zur Abtrennung des Stoffs von den Zellen der Probe. Zur Differenzierung zwischen apoptotischen und nekrotischen Zellen kann statt der Probe auch eine weitere aus der Zellsupension entnommene Probe verwendet werden. Dadurch kann vermieden werden, daß das Lektin mit dem Stoff in Kontakt kommt. Das ermöglicht den Einsatz eines Lektins, das mit dem Stoff so wechselwirkt, daß bei gleichzeitiger Anwesenheit des Stoffs und des Lektins die Schritte lit. c) oder lit. bb) beeinflußt werden würden. Die Schritte lit. c) und/oder bb) können mittels eines durchflußzytometrischen Verfahrens durchgeführt werden.

Weiterhin betrifft die Erfindung einen Kit zur Durchführung des erfindungsgemäßen Verfahrens enthaltend ein mit einem Fluorophor markiertes Lektin und eine zur Durchführung des Schritts lit. b) geeignete Säure. Die Säure kann Ameisensäure oder auch Essigsäure sein. Vorteilhafterweise ist das Lektin in der Säure oder einer im Saueren gepufferten Lösung gelöst. Darüber hinaus kann in dem Kit ein zum Erhöhen des pH-Werts der angesäuerten Probe geeignetes Agens enthalten sein. Das Agens ist vorzugsweise ein Puffer, insbesondere ein Phosphatpuffer, oder eine alkalische Lösung, insbesondere eine Carbonatlösung. In einer Ausgestaltung ist in dem Kit auch ein zur Fixierung der Zellen geeignetes Agens, insbesondere Paraformaldehyd, enthalten.

Nachfolgend werden Ausführungsbeispiele der Erfindung anhand der Zeichnung beschrieben. Hierin zeigen
- Fig.1: den Anteil positiver bzw. gefärbter peripherer Blutlymphozyten nach UV-B-Bestrahlung mit 120 mJ/cm² und Färbung mit Propidiumiodid, FITC-markiertem Annexin V oder dem erfindungsgemäßen Verfahren unter Verwendung von FITC-markiertem Narcissus pseudonarcissus als Funktion der Zeit,
- Fig.2: die Zahl positiver peripherer Blutlymphozyten als Funktion der Fluoreszenzintensität,
- Fig.3: den Anteil positiver peripherer Blutlymphozyten nach Behandlung mit Staurosporin und Färbung mit Propidiumiodid, FITC-markiertem Annexin V oder dem erfindungsgemäßen Verfahren unter Verwendung verschiedener FITC-markierter Lektine als Funktion der Zeit,
- Fig.4a,b: einen Vergleich verschiedener Nachweisverfahren für Apoptose anhand von mit 60 und 240 mJ/cm² bestrahlten Ramos-Zellen,
- Fig.5a,b: einen Vergleich verschiedener Nachweisverfahren für Apoptose anhand von mit 60 und 240 mJ/cm² bestrahlten Jurkat-Zellen,
- Fig.6a,b: einen Vergleich verschiedener Nachweisverfahren für Apoptose anhand von mit 60 und 240 mJ/cm² bestrahlten NALM 6-Zellen,
- Fig.7a,b: einen Vergleich verschiedener Nachweisverfahren für Apoptose anhand von mit 60 und 240 mJ/cm² bestrahlten Raji-Zellen,
- Fig.8a,b: einen Vergleich verschiedener Nachweisverfahren für Apoptose anhand von mit 60 und 240 mJ/cm² bestrahlten 697-Zellen,
- Fig.9a,b: einen Vergleich verschiedener Nachweisverfahren für Apoptose anhand von mit 60 und 240 mJ/cm² ARH 77-Zellen,
- Fig. 10a, b: einen Vergleich verschiedener Nachweisverfahren für Apoptose anhand von mit 60 und 240 mJ/cm² bestrahlten BALL-Zellen,
- Fig.11: einen Vergleich der Stabilität von mit Lektin und Annexin V hergestellten Färbungen für mit 60 mJ/cm² bestrahlte Zellinien,
- Fig.12: einen Vergleich der Stabilität von mit Lektin und Annexin V hergestellten Färbungen von mit 240 mJ/cm² bestrahlten Zellinien und
- Fig.13: den Einfluß der Säurebehandlung auf das erfindungsgemäße Verfahren.

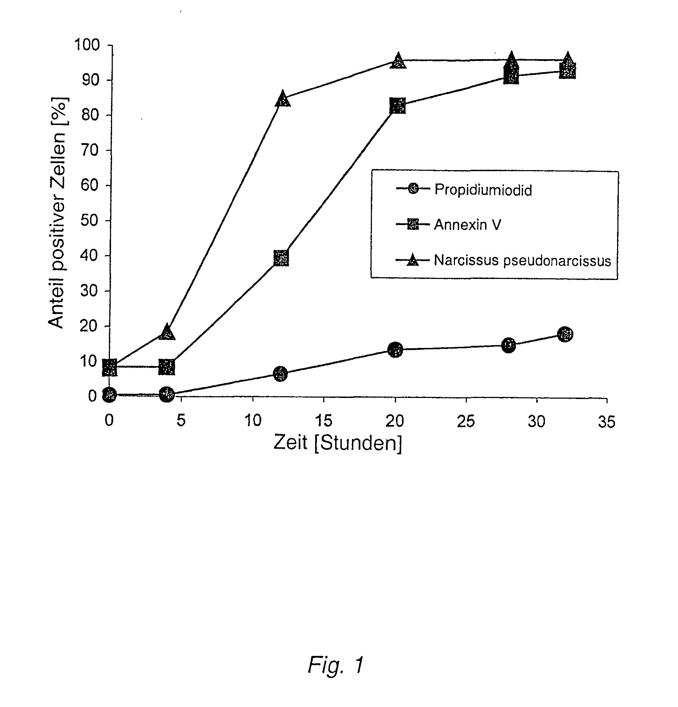

Die Versuche, deren Ergebnisse Fig. 1 bis Fig. 3 zu Grunde liegen, sind mit einer Suspension von aus Vollblut isolierten peripheren Blutlymphozyten durchgeführt worden. Zur Auslösung von Apoptose sind die Zellen mit Staurosporin in einer Endkonzentration von 2 µM oder mit UV-B-Strahlung behandelt worden. Für die UV-B-Behandlung ist eine Suspension der Zellen in einer flachen Schale ausgebreitet und mit 120 mJ/cm² bestrahlt worden. Zu 100 µl der Zellsuspension mit ca. 50.000 Zellen sind je 4 µg der FITC-markierten Lektine Phaseolus limensis, Arachis hypogea, Maackia amurensis oder Narcissus pseudonarcissus bzw. 2 µg FITC-markiertes Ulex europaeus I oder Ulex europaeus II zugesetzt worden. Alternativ sind zu 100 µl der Zellsuspension FITC-markiertes Annexin V zugesetzt worden, wie von der Herstellerfirma Boehringer Mannheim GmbH angegeben. Die unterschiedlich behandelten Zellsuspensionen sind eine halbe Stunde bei 4°C inkubiert worden. Erfindungsgemäß sind Lektin-behandelte Zellsuspensionen anschließend unter intensivem Mischen 10 Sekunden mit 600 µl Säure, vorzugsweise 0,12 Vol. % Ameisensäure, behandelt worden. Anschließend erfolgte eine Neutralisation des pH-Werts, vorzugsweise durch 265 µl einer Na₂CO₃ (0,6 Gew. %)-, NaCl (1,45 Gew. %)-, Na₂SO₄ (3,13 Gew. %)- Lösung, ebenfalls unter 10 Sekunden intensiver Mischung. Die Fixierung der Zellen erfolgte, vorzugsweise unter intensivem Mischen, mit 100 µl Paraformaldehyd in einer Konzentration von 1 Gew. %. Alternativ können die Lektin behandelten Zellsuspensionen dem Q-Prep®- Verfahren der Firma Coulter im 35 Sekunden Zyklus unterworfen werden. Ein nicht mit dem erfindungsgemäßen Verfahren behandelter Teil der Zellsuspension ist nach der Apoptose-auslösenden Behandlung für 30 Minuten mit Propidiumiodid in einer Konzentration von 1 µg/ml behandelt worden. Die Analyse aller Zellen erfolgte mit Hilfe der Durchflußzytometrie.

Fig. 1 zeigt den prozentualen Anteil peripherer Blutlymphozyten, die durch die Behandlung mit Propidiumiodid, FITC-markiertem Annexin V und dem erfindungsgemäßen Verfahren unter Verwendung von FITC-markiertem Narcissus pseudonarcissus-Lektin in Abhängigkeit von der Zeit nach einer UV-B-Bestrahlung gefärbt worden sind. Apoptotische Zellen werden auch mehr als 30 Stunden nach der UV-B-Bestrahlung kaum durch Propidiumiodid gefärbt. Das zeigt, daß die Membran apoptotischer Zellen im wesentlichen intakt ist. Der Anteil Narcissus pseudonarcissus-Lektin-gefärbter Zellen steigt früher und stärker an als der Anteil Annexin V-gefärbter Zellen. Die Färbung mit Narcissus pseudonarcissus-Lektin erlaubt bereits 5 - 10 Stunden nach der Auslösung der Apoptose durch UV-B-Bestrahlung eine zuverlässige Detektion apoptotischer Zellen.

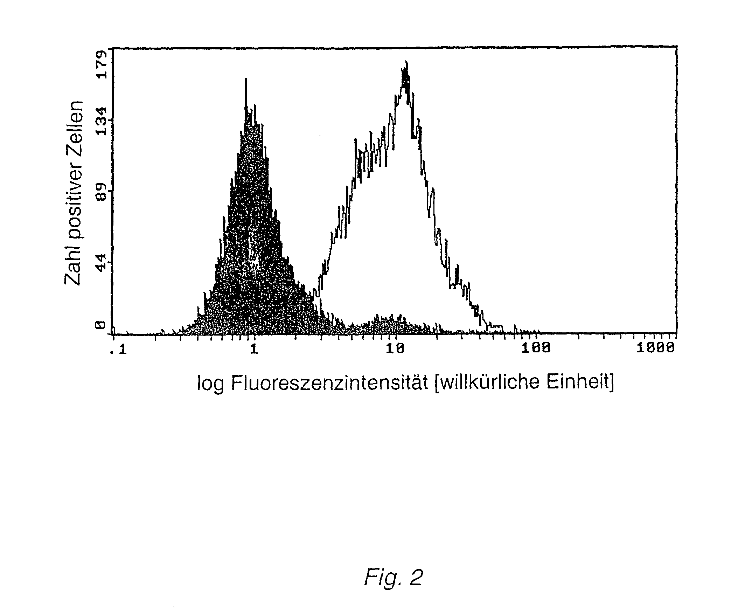

Fig. 2 zeigt die Zahl gefärbter peripherer Blutlymphozyten in Abhängigkeit von der Fluoreszenzintensität nach Durchführung des erfindungsgemäßen Verfahren unter Verwendung von FITC-markiertem Narcissus pseudonarcissus-Lektin. Das nicht ausgefüllte Feld unter der durchgezogenen Linie repräsentiert Zellen, die mit einer Dosis von 120 mJ/cm² mit UV-B behandelt und 24 Stunden später dem erfindungsgemäßen Verfahren unterworfen worden sind. Das schwarze Feld repräsentiert Zellen, die nicht bestrahlt, sonst aber gleich behandelt worden sind. Die Apoptose der Zellen führt zu einer deutlichen Zunahme der Fluoreszenzintensität. Die durch den rechten Teil des schwarzen Felds repräsentierte hohe Fluoreszenzintensität einiger unbehandelter Zellen resultiert aus auch in der Zellsuspension der unbehandelten peripheren Blutlymphozyten zu einem geringen Anteil vorhandenen apoptotischen Zellen.

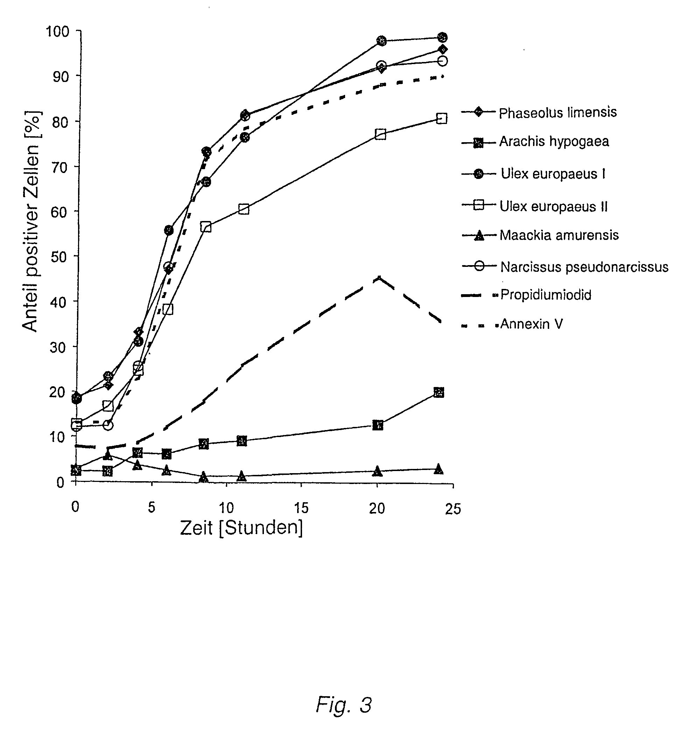

Fig. 3 zeigt den prozentualen Anteil peripherer Blutlymphozyten, die durch die Behandlung mit Propidiumiodid, FITC-markiertem Annexin V und mit dem erfindungsgemäßen Verfahren unter Verwendung verschiedener Lektine in Abhängigkeit von der Zeit nach dem Auslösen einer Apoptose durch Staurosporin gefärbt worden sind. Die Behandlung der Zellen mit Staurosporin führt zu einem deutlichen Anstieg des Anteils Propidiumiodid-gefärbter Zellen. Das zeigt, daß die Staurosporin-Behandlung auch zur Bildung nekrotischer Zellen führt. Mittels des Sialylsäure-bindenden Lektins Maackia amurensis und des Lektins Arachis hypogaea läßt sich keine wesentliche Färbung der Zellen erreichen. Die Färbung mit Phaseolus limensis, Ulex europaeus I, Ulex europaeus II, Narcissus pseudonarcissus und Annexin V führt zu einem deutlichen Anstieg des Anteils gefärbter Zellen nach der Staurosporin-Behandlung.

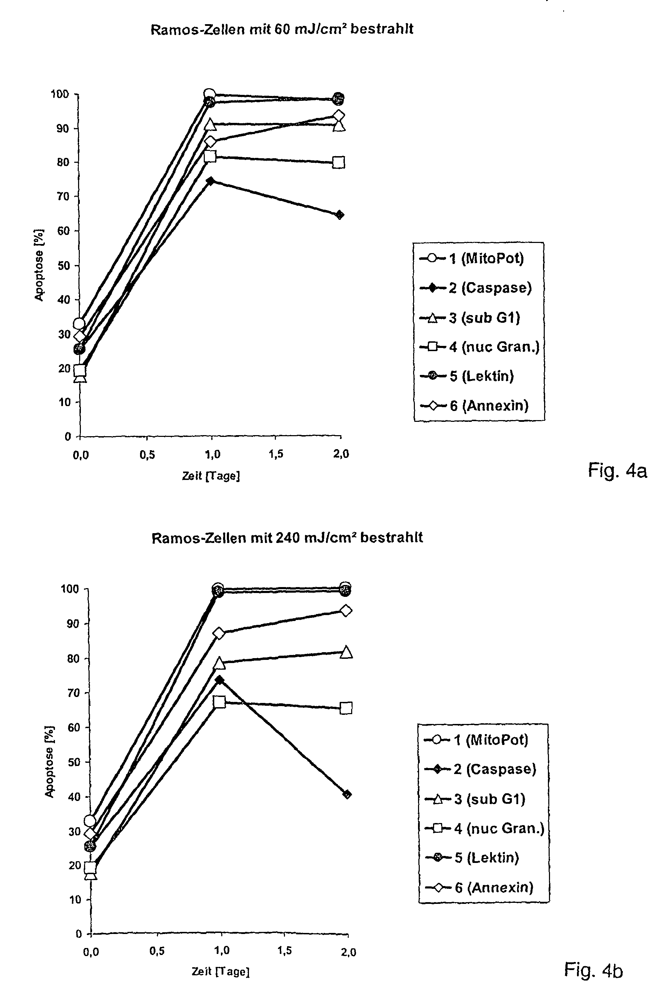

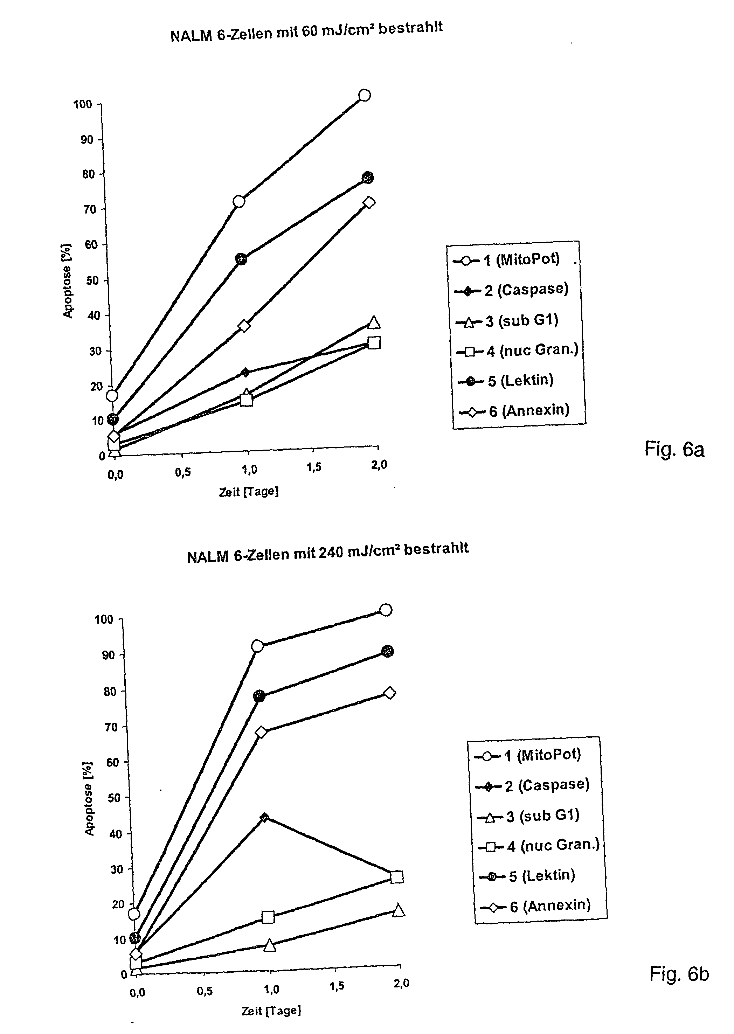

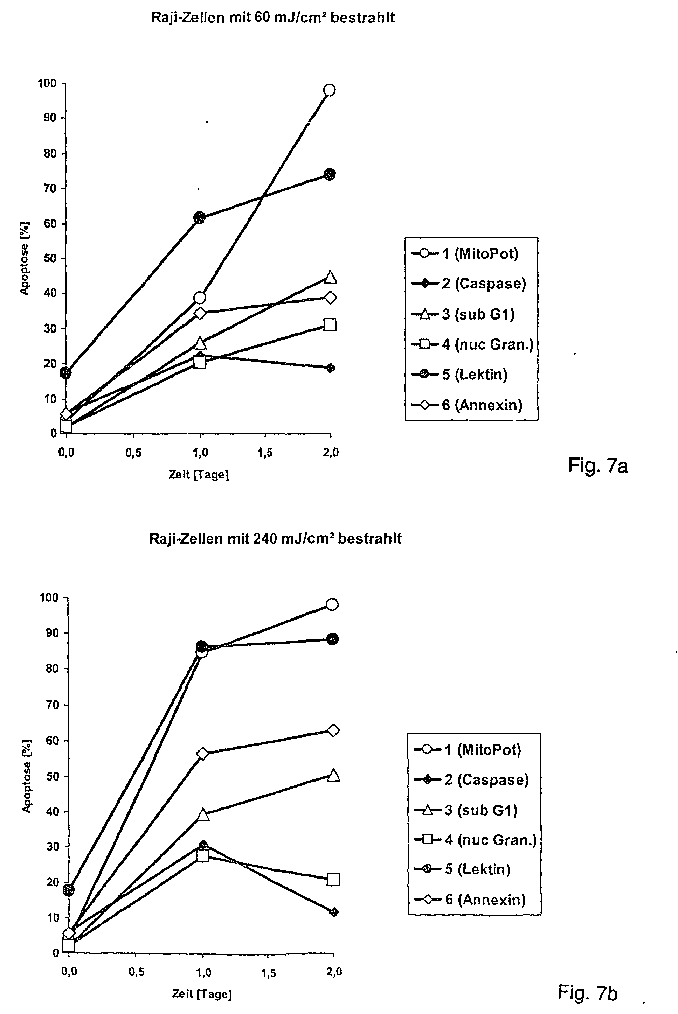

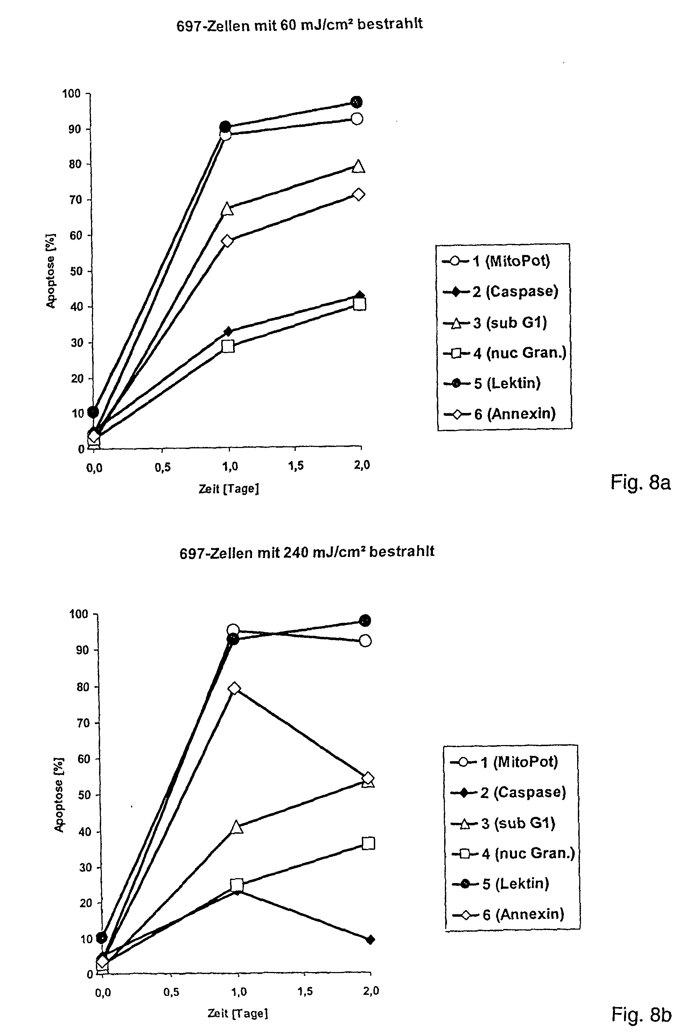

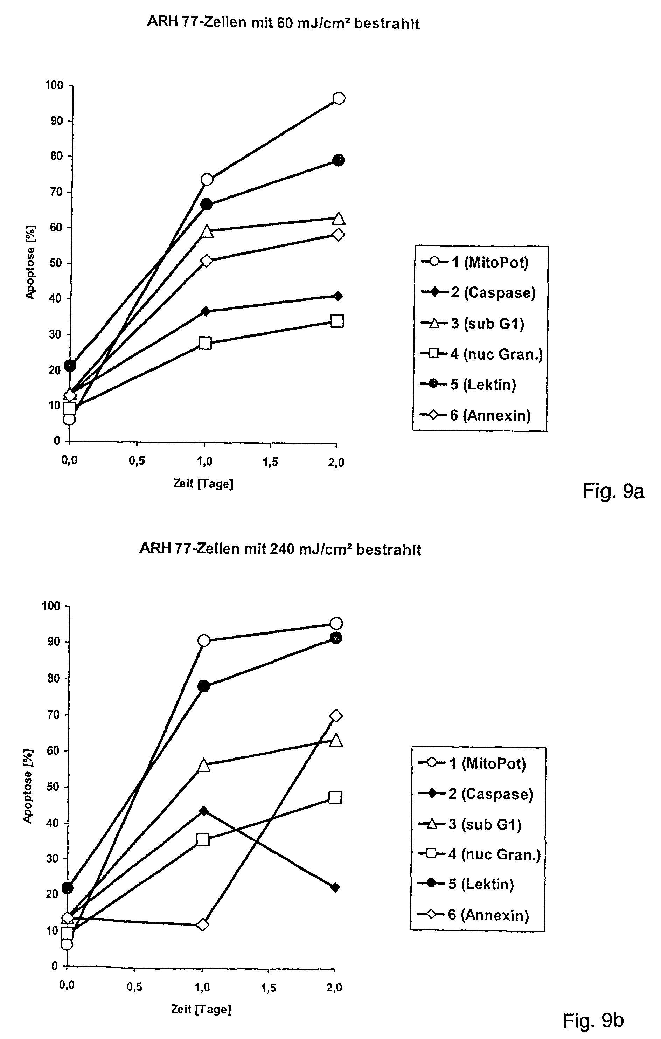

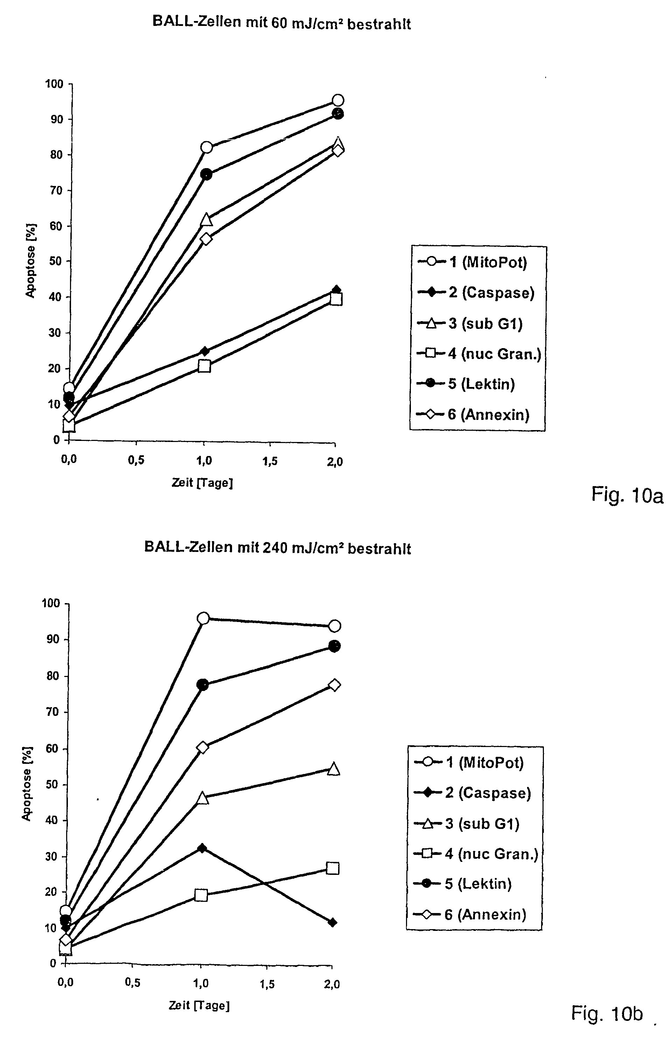

In den Fig. 4 bis 10 sind Ergebnisse eines Vergleichs verschiedener Methoden zur Apoptoseanalyse mit humanen Zellinien gezeigt. Für den Fachmann ist klar, daß der Anmeldungsgegenstand auch mit nicht-humanen Zellen durchführbar ist.

Es sind die folgenden Zellinien verglichen worden:

| | | |
|---|---|---|
| Zellinie | Beschreibung der Zellinie | ATCC-Bezeichnung |
| Ramos | lymphoblastoides B-Zell-Lymphom (Burkitt Lymphom) | CRL-1596 |
| Jurkat | T-Zellinie (T-ALL) | TIB-152 |
| NALM 6 | akute lymphoblastische Prä-B-Leukämie | |
| Raji | lymphoblastisches B-Zell-Lymphom (Burkitt Lymphom) | CCL-86 |
| 697 | B-Zellinie | |
| ARH 77 | lymphoblastische B-Zellinie | CRL-1621 |
| BALL | B-Zell-Lymphom | |

Zellinien wurden vor den jeweiligen Analysen auf eine Dichte von 10⁶ Zellen/ml in Kulturmedium eingestellt. Pro Färbung wurden 100 µl (entsprechend ca. 10⁵ Zellen) dieser Suspension in Durchflußzytometer-Röhrchen vorgelegt.

### 1. Bestimmung des Mitochondrienpotentials mit DiOC₆(3) (3,3'-Dihexyloxacarbocyanin-iodid) ("MitoPot")

Zu den Zellen wurden 500 µl einer frisch bereiteten 10⁶ fachen Verdünnung von DiOC₆(3) (Sigma, München) mit Ringer-Lösung aus einer 40 mM Stammlösung in Dimethylsulfoxid gegeben. Dieser Ansatz wurde 30 min bei 4°C inkubiert und anschießend innerhalb von 1 h im Durchflußzytometer (FL-1) vermessen. Als apoptotisch wurden diejenigen Zellen gewertet, die ein reduziertes Membranpotential aufwiesen (reduzierte FL-1-Werte).

### 2. Nachweis der Caspase-Aktivität durch FITC-markiertes zVAD-FMK (Carbobenzoxy-Valyl-Alanyl-Aspartyl-[O-Methyl]-Fluoromethylketon) ("Caspase")

Zu den Zellen wurden 2 µl einer 100fachen Verdünnung von FITC-markiertem zVAD-FMK (CaspACE™, Promega, Mannheim) in Zellkulturmedium gegeben. Dieser Ansatz wurde 60 min bei 37°C inkubiert, anschließend mit 2 ml PBS versetzt und weitere 10 min bei Raumtemperatur inkubiert. Danach wurde 5 min bei 1.600 rpm zentrifugiert. Der Überstand wurde verworfen, das Sediment in 500 µl PBS mit 1% Paraformaldehyd resuspendiert und anschließend innerhalb von 4 h im Durchflußzytometer analysiert. Als apoptotisch wurden diejenigen Zellen gewertet, die eine erhöhte Caspaseaktivität aufwiesen (erhöhte FL-1-Werte)

### 3. Nachweis von Zellen mit sub-G1 DNA-Gehalt ("sub G1")

Die Zellen wurden mit 500 µl Färbelösung versetzt und 24 h bei 4°C inkubiert. Die Färbelösung besteht aus 500 ml deionisiertem Wasser, das mit 500 µl Triton X-100, 500 mg Natriumcitrat sowie 1-10 µg Propidiumiodid versetzt wird. Die benötigte Menge Propidiumiodid wird für jeden Ansatz individuell ermittelt. Bei den dargestellten Meßwerten betrug die Propidiumiodid-Konzentration der Färbelösung 2,3 µg/ml. Die fertige Färbelösung ist bei 4°C bis zu 4 Wochen stabil. Nach der 24stündigen Inkubation wurden die Zellen innerhalb von 12 h im Durchflußzytometer (FL-4) vermessen. Als spät-apoptotisch wurden diejenigen Zellen gewertet, die einen sub-G1 DNA-Gehalt aufwiesen (erniedrigte FL-4-Werte).

### 4. Nachweis von Zellen mit granulären Kernen ("nuc Gran.")

Der Ansatz ist der gleiche wie beim Nachweis von Zellen mit sub-G1 DNA-Gehalt. Jedoch wurden diejenigen Zellen als apoptotisch gewertet, die bei normalem DNA-Gehalt erhöhte "forward" und "side scatter" aufwiesen.

### 5. Nachweis der Bindung von Narcissus pseudonarcissus Lektin an säurebehandelte Zellen ("Lektin")

Die Zellen wurden mit 10 µl FITC-markiertem Lektin von Narcissus pseudonarcissus (200 ng/µl in Ringer-Lösung) versetzt und anschließend 30 min bei 4°C inkubiert. Die Markierung von Lektin aus Narcissus pseudonarcissus erfolgte mit Hilfe des FluoroTag™ FITC Conjugation Kits (beide von Sigma, München). Nach der Inkubation wurde die Zellsuspension unter intensivem Mischen 10 s mit 600 µl Säure, vorzugsweise 0,12 Vol. % Ameisensäure, behandelt. Statt Ameisensäure kann auch eine 1 %ige Essigsäure verwendet werden. Anschließend erfolgte eine Anhebung des pH-Werts, vorzugsweise durch 265 µl einer Lösung aus 0,6 Gew. % Na₂CO₃, 1,45 Gew. % NaCl und 3, 13 Gew. % Na₂SO₄ , ebenfalls unter 10 s intensiver Mischung. Statt der vorgenannten Lösung kann auch ein Carbonatpuffer, ph 9,5, hergestellt durch Lösen von 22,43 g NaHCO₃ und 14,15 g Na₂CO₃ in 500 ml Wasser, verwendet werden. Die Fixierung der Zellen erfolgte dann durch Zugabe von 100 µl Paraformaldehyd in einer Konzentration von 1 Gew. % unter intensiver Mischung. Die Fixierung kann auch durch eine Ringer-Lösung erfolgen, die 4 % Formaldehyd enthält und der ferner 1 : 1000 eine 1 %ige Essigsäure zugesetzt ist. Die Messung der Zellen erfolgte dann innerhalb von 12 h im Durchflußzytometer. Als tot (apoptotisch oder nekrotisch) wurden diejenigen Zellen gewertet, die nach Säurebehandlung eine erhöhte Lektinbindung (erhöhte FL-1-Werte) und/oder einen erniedrigten "forward scatter" aufwiesen. Der Wert der apoptotischen Zellen ergab sich, indem von der Zahl der toten Zellen die Zahl der Propidiumiodidpositiven, nekrotischen Zellen, die in einer separaten Messung (s. 6.) ermittelt wurde, abgezogen wurden.

### 6. Nachweis der Exposition von Phosphatidylserin durch Bindung von Annexin V ("Annexin")

Die Zellen wurden mit 500 µl Färbelösung versetzt und anschließen 30 min bei 4°C inkubiert. Die Färbelösung enthält 1 µl FITC-markiertes Annexin V (200 µg/ml) und Propidiumiodid in individuell ermittelter Menge (10-100 ng) in 1 ml Ringer-Lösung. Bei den dargestellten Meßwerten betrug die Propidiumiodid-Konzentration der Färbelösung 60 ng/ml. Die fertige Färbelösung ist bei 4°C bis zu 2 Wochen stabil. Die Markierung von humanem Annexin V (Roche, Mannheim) erfolgte mit Hilfe des FluoroTag™ FITC Conjugation Kits (Sigma, München.

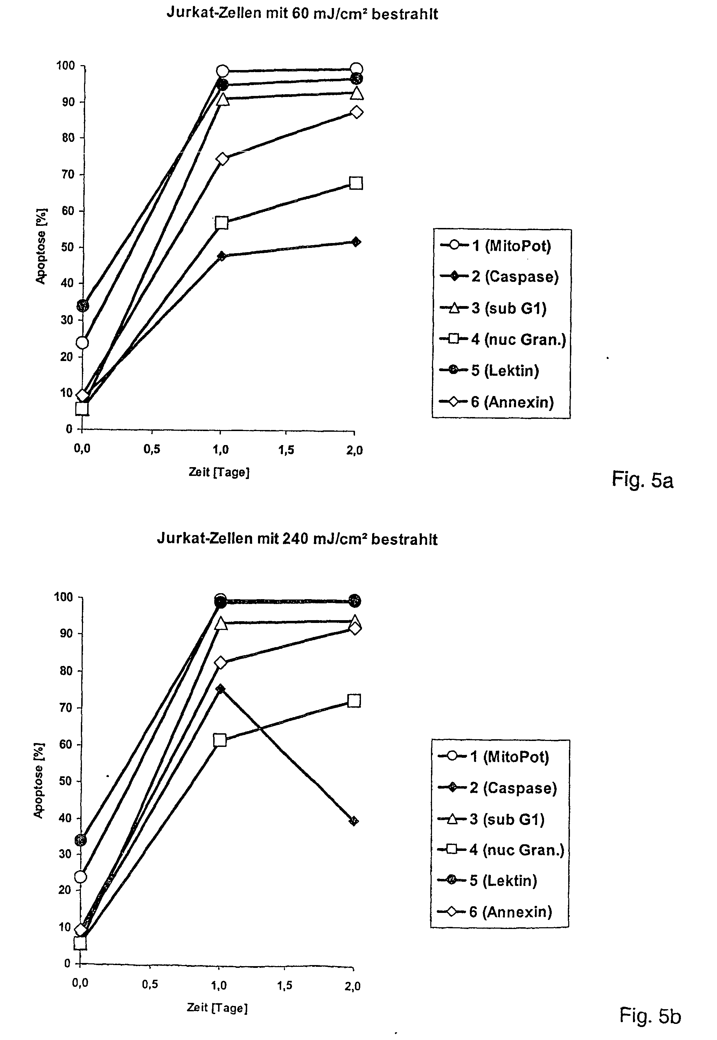

In den Fig. 4 bis 10 sind im Vergleich die Ergebnisse der mit den unterschiedlichen Färbeverfahren erzielten Färbungen gezeigt. Sämtliche Verfahren zum Nachweis der Apoptose sind zeitabhängig. Bei einigen Zellinien und Bestrahlungsdosen ist das erfindungsgemäße Verfahren und Verwendung von Lektin und Säurebehandlung besonders schnell. Es wird auf die in den Fig. 5, 7, 8 und 9 gezeigten Ergebnisse verwiesen. Das erfindungsgemäße Verfahren zeigt hier sofort ein schnelles Ansprechen beim Vorliegen apoptotischer Zellen. Der Nachweis von Veränderungen der Zellmembran bei der Apoptose mit Hilfe des erfindungsgemäßen Verfahrens ist bei allen untersuchten Zellen schneller als der nach dem heutigen Stand der Technik zumeist zu diesem Zweck eingesetzte Nachweis mit Annexin V.

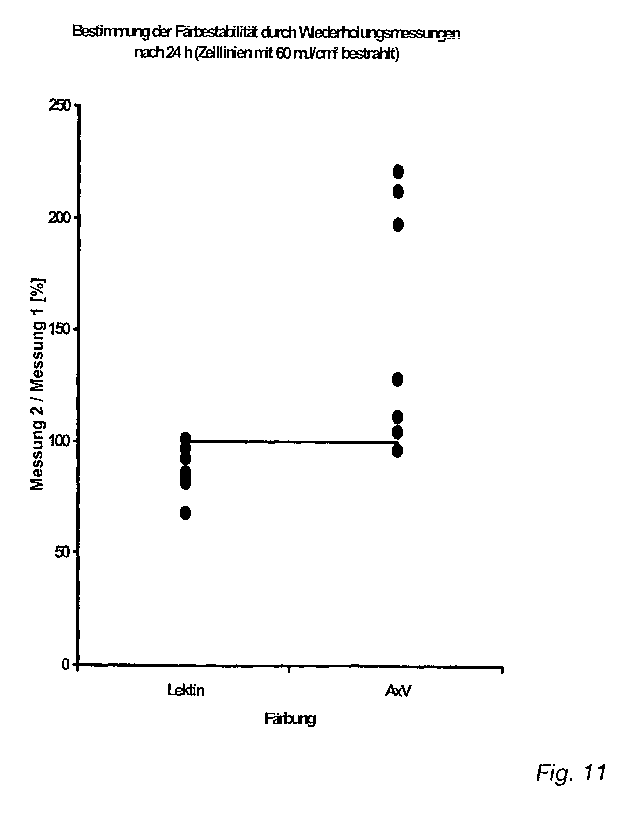

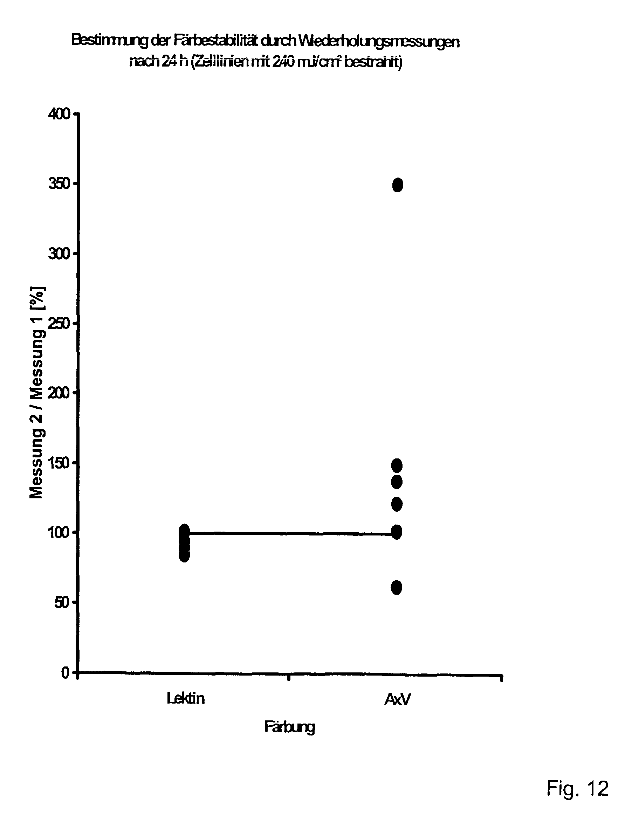

In den Fig. 11 und 12 ist ein Vergleich der Apoptose-Färbungen bei Wiederholungsmessungen nach 24 Stunden für mit 60 und 240 mJ/cm² bestrahlte Zellinien gezeigt. Die Zellen wurden 24 Stunden nach der Bestrahlung gefärbt und sofort gemessen. Anschließend wurden die Zellen bei 4°C für weitere 24 Stunden aufbewahrt und nochmals gemessen. Es zeigte sich, daß die säurebehandelten und mit Lektin gefärbten Zellen eine wesentlich stabilere Färbung aufwiesen als die mit Annexin V gefärbten Zellen. Beim erfindungsgemäßen Verfahren ist es also nicht notwendig, sofort nach der Färbung auch die Messung durchzuführen. Das vorgeschlagene Verfahren ist leichter durchführbar und weniger fehleranfällig.

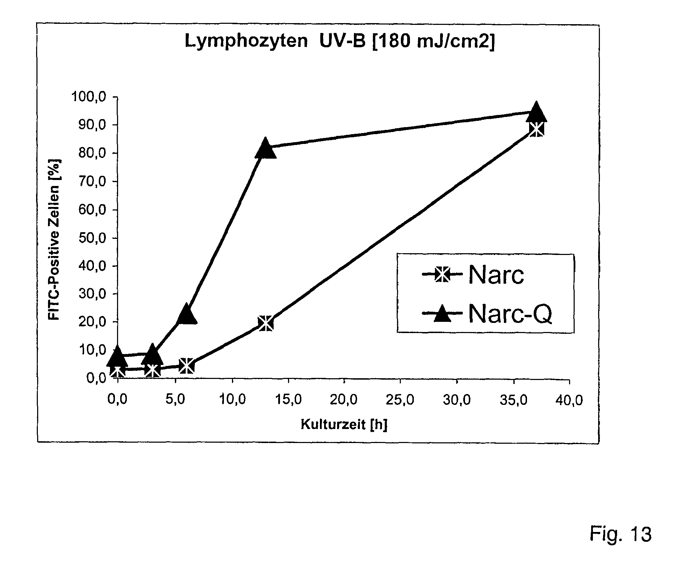

Fig. 13 zeigt den Einfluß der Säurebehandlung auf das erfindungsgemäße Verfahren. Die Proben wurden wie unter Ziff.5 beschrieben hergestellt, wobei nur die mit "Narc Q" bezeichneten Proben mit Säure behandelt wurden, während die mit "Narc" bezeichneten Proben nicht mit Säure behandelt wurden. Die Messung erfolgte jeweils im Durchflußzytofluorometer. Die Ergebnisse zeigen klar, daß der Nachweis infolge der Säurebehandlung erheblich schneller erfolgen kann.

## Patentansprüche

1. Verfahren zum Nachweis apoptotischer und nekrotischer Zellen in einer Zellsuspension mit den folgenden Schritten:
a) Inkontaktbringen einer der Zellsuspension entnommenen Probe mit einem Lektin, welches dazu geeignet ist, an in der Membran apoptotischer und nekrotischer Zellen enthaltene Zuckerstrukturen zu binden,
b) Ansäuern der Probe, wobei eine solche Menge an Säure zugegeben wird, daß **dadurch** die äußere Zellmembran apoptotischer Zellen permeabilisiert wird, während die Zellmembran vitaler Zellen nicht permeabilisiert wird und
c) Detektieren der Menge des an apoptotische und nekrotische Zellen gebundenen Lektins.

2. Verfahren nach Anspruch 1, wobei der Probe beim Schritt lit. b) Ameisensäure zugesetzt wird.

3. Verfahren nach Anspruch 1 oder 2, wobei der pH-Wert der angesäuerten Probe nach den Schritten lit. a) und lit. b) und vor dem Schritt lit. c) erhöht wird.

4. Verfahren nach Anspruch 3, wobei der angesäuerten Probe zum Erhöhen des pH-Werts ein Puffer, insbesondere ein Phosphatpuffer, oder eine alkalische Lösung, insbesondere eine Carbonat lösung , zugesetzt wird.

5. Verfahren nach einem der vorhergehenden Ansprüche, wobei die Zuckerstrukturen Mannosyl-, insbesondere α-D-Mannosyl-, Glucosyl-, insbesondere α-D-Glucosyl-, Glucosamin-, insbesondere N-Acetylglucosamin, Galactosyl-, Galactosamin-, insbesondere N-Acetylgalactosamin-, Fucosyl-, insbesondere L-Fucosyl-, oder N,N'-Diacetylchitobiose-Reste enthalten.

6. Verfahren nach einem der vorhergehenden Ansprüche, wobei das Lektin Pisum sativum, Phaseolus limensis, Ulex europaeus I, Ulex europaeus II, Narcissus pseudonarcissus, Concanavalin A oder Ricinus communis ist.

7. Verfahren nach einem der vorhergehenden Ansprüche, wobei das Lektin eine Markierungssubstanz aufweist.

8. Verfahren nach einem der vorhergehenden Ansprüche, wobei die Markierungssubstanz ein Fluorophor, insbesondere Fluoresceinisothiocyanat, ist.

9. Verfahren nach einem der vorhergehenden Ansprüche, wobei im unmittelbaren Anschluß an die Schritte lit. a) und b) oder das Erhöhen des pH-Werts eine Fixierung der Zellen erfolgt.

10. Verfahren nach Anspruch 9, wobei die Fixierung mittels Paraformaldehyd erfolgt.

11. Verfahren nach einem der vorhergehenden Ansprüche, wobei zur Differenzierung zwischen apoptotischen und nekrotischen Zellen die folgenden Schritte durchgeführt werden:
aa) Inkontaktbringen der Probe mit einem Stoff zur Anfärbung des Zellinneren, insbesondere des Zellkerns, welcher die äußere Zellmembran apoptotischer Zellen nicht oder langsam durchdringt und
bb) Detektieren der Menge des in das Innere der Zellen eingedrungenen Stoffs,
wobei Schritt lit. aa) vor den Schritten lit. a) und b) durchgeführt wird und dazwischen der überschüssige Stoff zur Anfärbung des Zellinneren durch Waschen entfernt wird.

12. Verfahren nach Anspruch 11, wobei der Stoff Propidiumiodid oder Trypanblau ist.

13. Verfahren nach Anspruch 11 oder 12, wobei zwischen den Schritten lit. aa) und lit. bb) ein Waschschritt zur Abtrennung des Stoffs von den Zellen der ersten Probe erfolgt.

14. Verfahren nach einem der vorhergehenden Ansprüche, wobei die Schritte lit. c) und/oder lit. bb) mittels eines durchflußzytometrischen Verfahrens durchgeführt werden.

15. Kit zur Durchführung des erfindungsgemäßen Verfahrens nach einem der Ansprüche 1 bis 14 enthaltend ein mit einem Fluorophor markiertes Lektin und eine zur Durchführung des Schritts lit. b) geeignete Säure.

16. Kit nach Anspruch 15, wobei die Säure Ameisensäure ist.

17. Kit nach Anspruch 15 oder 16, wobei das Lektin in der Säure oder einer im Saueren gepufferten Lösung gelöst ist.

18. Kit nach einem der Ansprüche 15 bis 17, wobei ein zum Erhöhen des pH-Werts der angesäuerten Probe geeignetes Agens enthalten ist.

19. Kit nach Anspruch 18, wobei das Agens ein Puffer, insbesondere ein Phosphatpuffer, oder eine alkalische Lösung, insbesondere eine Carbonatlösung, ist.

20. Kit nach einem der Ansprüche 15 - 19, wobei ein zur Fixierung der Zellen geeignetes Agens, insbesondere Paraformaldehyd, enthalten ist.

## Claims

1. Method for the detection of apoptotic and necrotic cells in a cell suspension with the following steps:
a) Bringing a sample taken from the cell suspension into contact with a lectin which is suitable for bonding to sugar structures contained in the membrane of apoptotic and necrotic cells,
b) Acidulating of the sample, wherein such an amount of acid is added so that the exterior cell membrane of apoptotic cells is made permeable while the cell membrane of vital cells is not made permeable and
c) Detection of the amount of lectin bound to the apoptotic and necrotic cells.

2. Method as defined in claim 1, wherein formic acid is added to the sample during step letter b).

3. Method as defined in claim 1 or 2, wherein the pH value of the acidulated sample is increased after steps letter a) and letter b) and before step letter c).

4. Method as defined in claim 3, wherein a buffer, in particular a phosphate buffer, or an alkaline solution, in particular a carbonate solution, is added to the acidulated sample to increase the pH value.

5. Method as defined in one of the preceding claims, wherein the sugar structures contain mannosyl-, in particular α-D mannosyl-, glucosyl-, in particular α-D glucosyl-, glucosamine-, in particular N-acetylglucosamine, galactosyl-, galactosamine-, in particular N-acetylgalactrosamine-, fucosyl-, in particular L-fucosyl-, or N,N' diacetylchitobiose-residues.

6. Method as defined in one of the preceding claims, wherein the lectin is pisum sativum, phaseolus limensis, ulex europaeus I, ulex europaeus II, narcissus pseudonarcissus, concanavalin A or ricinus communis.

7. Method as defined in one of the preceding claims, wherein the lectin has a tagging substance.

8. Method as defined in one of the preceding claims, wherein the tagging substance is a fluorophore, in particular fluoresceinisothiocyanate.

9. Method as defined in one of the preceding claims, wherein a fixation of the cells occurs immediately after the steps letter a) and b) or after the increase of the pH value.

10. Method as defined in claim 9, wherein the fixation is performed via paraformaldehyde.

11. Method as defined in one of the preceding claims, wherein the following steps are performed to differentiate between apoptotic and necrotic cells:
aa) Bringing the sample into contact with a substance for intracellular staining, in particular the cell nucleus, which does not penetrate or only slowly penetrates the exterior cell membrane of apoptotic cells and
bb) Detection of the amount of the substance which penetrated the interior of the cells,
wherein step letter aa) is performed before steps letter a) and b) and in between the excess substance for intracellular staining is removed by rinsing.

12. Method as defined in claim 11, wherein the substance is propidiumiodide or trypan blue.

13. Method as defined in claim 11 or 12, wherein a rinsing step to separate the substance from the cells of the first sample occurs between the steps letter aa) and letter bb).

14. Method as defined in one of the preceding claims, wherein the steps letter c) and/or letter bb) are performed via a flow cytometric method.

15. Kit for execution of the method provided by the invention as defined in one of the claims 1 to 14 containing a lectin tagged with a fluorophore and an acid suitable for the execution of step letter b).

16. Kit as defined in claim 15, wherein the acid is formic acid.

17. Kit as defined in claim 15 or 16, wherein the lectin is dissolved in the acid or a solution buffered in acid.

18. Kit as defined in one of the claims 15 to 17, wherein is contained an agent suitable for the increase of the pH value of the acidulated sample.

19. Kit as defined in claim 18, wherein the agent is a buffer, in particular a phosphate buffer, or a alkaline solution, in particular a carbonate solution.

20. Kit as defined in one of the claims 15 to 19, wherein is contained a agent suitable for the fixation of the cells, in particular paraformaldehyde.

## Revendications

1. Procédé de détection de cellules apoptotiques et nécrotiques dans une suspension cellulaire, comportant les étapes suivantes :
a) la mise en contact d'un échantillon prélevé de la suspension cellulaire avec une lectine qui est appropriée pour se lier à des structures sucre contenues dans la membrane des cellules apoptotiques et nécrotiques,
b) l'acidification de l'échantillon, où on ajoute une quantité d'acide telle que de ce fait, la membrane cellulaire externe des cellules apoptotiques est rendue perméable, alors que la membrane cellulaire des cellules vitales n'est pas rendue perméable, et
c) la détection de la quantité de la lectine liée aux cellules apoptotiques et nécrotiques.

2. Procédé selon la revendication 1, dans lequel on ajoute un acide formique à l'échantillon lors de l'étape b).

3. Procédé selon la revendication 1 ou 2, dans lequel la valeur de pH de l'échantillon acidifié est augmentée après l'étape a) et l'étape b) et avant l'étape c).

4. Procédé selon la revendication 3, dans lequel, pour augmenter la valeur du pH, on ajoute à l'échantillon acidifié un tampon, en particulier un tampon de phosphate, ou une solution alcaline, en particulier une solution de carbonate.

5. Procédé selon l'une des revendications précédentes, dans lequel les structures sucre contiennent des radicaux mannosyl-, en particulier, α-D-mannosyl-, glucosyl-, en particulier α-D-glucosyl-, glucosamine-, en particulier N-acétylglucosamine, galactosyl-, galactosamine-, en particulier N-acétylgalactosamine, fucosyl-, en particulier L-fucosyl-, ou N,N-diacétylchitobiose.

6. Procédé selon l'une des revendications précédentes, dans lequel la lectine est Pisum sativum, Phaseolus limensis, Ulex europaeus I, Ulex europaeus II, Narcissus pseudonarcissus, Concanavalin A ou Ricinus communis.

7. Procédé selon l'une des revendications précédentes, dans lequel la lectine présente une substance de marquage.

8. Procédé selon l'une des revendications précédentes, dans lequel la substance de marquage est un fluorophore, en particulier un isothiocyanate de fluorescéine.

9. Procédé selon l'une des revendications précédentes, dans lequel on réalise une fixation des cellules directement après les étapes a) et b) ou après l'augmentation de la valeur de pH.

10. Procédé selon la revendication 9, dans lequel la fixation est réalisée au moyen d'un paraformaldéhyde.

11. Procédé selon l'une des revendications précédentes, dans lequel on réalise les étapes suivantes pour effectuer la différenciation entre cellules apoptotiques et nécrotiques :
aa) la mise en contact de l'échantillon avec une matière permettant de colorer l'intérieur des cellules, en particulier le noyau cellulaire, qui ne pénètre pas la membrane cellulaire externe des cellules apoptotiques ou la pénètre lentement, et
bb) la détection de la quantité de la matière ayant pénétré à l'intérieur des cellules,
dans lequel l'étape aa) est réalisée avant les étapes a) et b), et dans lequel la matière en excès pour colorer l'intérieur des cellules est éliminée entre-temps par lavage.

12. Procédé selon la revendication 11, dans lequel la matière est de l'iodure de propidium ou du bleu trypane.

13. Procédé selon la revendication 11 ou 12, dans lequel entre les étapes aa) et bb), on réalise une étape de lavage permettant de séparer la matière des cellules du premier échantillon.

14. Procédé selon l'une des revendications précédentes, dans lequel les étapes c) et/ou bb) sont réalisées au moyen d'un procédé par cytométrie de flux.

15. Ensemble pour la réalisation du procédé en accord avec la présente invention selon l'une des revendications 1 à 14, contenant une lectine marquée par un fluorophore et un acide approprié pour réaliser l'étape b).

16. Ensemble selon la revendication 15, dans lequel l'acide est de l'acide formique.

17. Ensemble selon la revendication 15 ou 16, dans lequel la lectine est dissoute dans l'acide ou dans une solution tamponnée à l'acide.

18. Ensemble selon l'une des revendications 15 à 17, dans lequel est contenu un agent approprié pour augmenter la valeur de pH de l'échantillon acidifié.

19. Ensemble selon la revendication 18, dans lequel l'agent est un tampon, en particulier un tampon de phosphate, ou une solution alcaline, en particulier une solution de carbonate.

20. Ensemble selon l'une des revendications 15 à 19, dans lequel est contenu un agent approprié pour fixer les cellules, en particulier du paraformaldéhyde.
